# NEUE EUROPÄISCHE PATENTSCHRIFT

(11) **EP 2 594 495 B2**
(45) Veröffentlichungstag und Bekanntmachung des Hinweises auf die Entscheidung über den Einspruch: **08.06.2022**
(45) Hinweis auf die Patenterteilung: 11.01.2017
(21) Anmeldenummer: 12193692.6
(22) Anmeldetag: 21.11.2012
(51) Int. Cl.: B65B 55/08, B65B 43/46, A61L 2/08, B67C 7/00, G21K 5/08

(54) **Vorrichtung, Anlage und Verfahren mit innengreifendem Halteelement zur Behältersterilisation mittels Elektronenstrahlen**
Apparatus, system and method with internally gripping holding element for container sterilization by means of electron beams
Dispositif, système et méthode avec elément de support à accroche intérieure pour la stérilisation de récipients au moyens de faisceaux d'électrons

(30) Priorität: 21.11.2011 DE 102011055552
(43) Veröffentlichungstag der Anmeldung: 22.05.2013
(73) Patentinhaber: Krones AG, 93073 Neutraubling (DE)
(72) Erfinder: Knott, Josef, 93073 Neutraubling (DE); Scheuren, Hans, 93073 Neutraubling (DE)
(74) Vertreter: Bittner, Bernhard

(56) Entgegenhaltungen:
- EP-A1- 1 982 921
- EP-A1- 2 123 580
- EP-A1- 2 279 952
- WO-A1-2011/002380
- WO-A2-2008/129397
- DE-A1-102007 050 582
- DE-A1-102010 012 569
- DE-A1-102010 020 996
- DE-A1-102010 028 984
- FR-A1- 2 923 802
- US-A1- 2009 317 506
- US-A1- 2010 025 275
- US-A1- 2010 159 060
- US-B2- 6 773 224
- US-B2- 7 008 215
- US-B2- 7 674 424
- US-B2- 7 694 802

## Beschreibung

Die Erfindung betrifft eine Vorrichtung zum Sterilisieren von Behältnissen und insbesondere einer Außenwandung von Behältnissen mittels beschleunigter Ladungsträger mit einer Ladungsträgererquelle zum Erzeugen der Ladungsträger und mit einer Beschleunigungseinrichtung die Ladungsträger in Richtung einer Außenwandung des Behältnisses beschleunigt, wobei die Behältnisse mittels einer Transporteinrichtung gegenüber der Ladungsträgererquelle entlang eines vorgegebenen Transportpfades relativbeweglich sind und die Transporteinrichtung mindestens ein Halteelement zum Halten mindestens eines Behältnisses aufweist. Bei einem Behältnis kann es sich um jedes beliebige Behältnis handeln, bevorzugt handelt es sich jedoch um Flaschen insbesondere Kunststoffflaschen und/oder Vorformlinge.

Aus dem Stand der Technik ist bekannt, dass vor dem Abfüllen ein Sterilisationsprozess der bereits fertig gestellten Flaschen in einem dafür vorgesehen Reinraum durchgeführt wird. Dies ist für eine Vielzahl von Getränken erforderlich, da sie unter aseptischen Bedingungen abgefüllt werden müssen. Ein Vorteil dieser Methode ist, dass alle zuvor stattfindenden Prozesse wie die Preform-Herstellung, deren Transport, deren Erwärmung und deren Blasen zur Flasche in einer unsterilen Umgebung stattfinden können. Nachteilig dabei ist jedoch, dass es erforderlich ist, die fertiggestellte, relativ große Flasche sterilisieren zu müssen.

Im einfachsten Fall kann das Sterilisieren der Behältnisse vor der Befüllung mit sensiblem Füllgut beispielsweise dadurch erfolgen, dass das Füllgut selbst soweit erhitzt und heiß abgefüllt wird, dass durch dessen Hitze die Flascheninnenseite sterilisiert wird. Aufgrund besonderer Temperaturempfindlichkeit ist dies jedoch oft nicht möglich, oder ein ausreichender Sterilisationsgrad kann aufgrund zu geringer Temperaturen nicht erreicht werden. Insbesondere bei vielen Lebensmitteln, Pharmazeutika und anderen sensiblen Produkten ist dies der Fall. Daher sind Verfahren entwickelt worden, um das leere Behältnis vor dem Füllvorgang separat zu entkeimen und dieses in einem nachgeschalteten Prozessschritt unter aseptischen Bedingungen mit an anderer Stelle sterilisiertem Füllgut zu befüllen.

Neben dem Füllvorgang ist die Sterilisation eines zu befüllenden Behälters daher einer der zentralen Prozessschritte in einer aseptischen Abfüllanlage. Die möglichen Sterilisationsformen variieren hinsichtlich der Entkeimungsmitteln und der Prozessführung. Aus dem Stand der Technik ist eine Vielzahl von Sterilisationsverfahren bekannt, bei denen die abtötende Wirkung auf chemischen Prozessen beruht. Die Sterilität des leeren Behältnisses, welche z.B. durch chemische Desinfektionsmittel wie beispielsweise Peroxide, Persäuren oder andere erreicht wird, muss dabei über einen vergleichsweise langen Zeitraum, nämlich bis zur Befüllung des Gebindes und das anschließende Verschließen aufrecht erhalten werden. Bei diesem Verfahren werden die Behältnisse zunächst einem sogenannten Isolator zugeführt, in dem sie mit dem Desinfektionsmittel beaufschlagt werden. Dort wird das Desinfektionsmittel eine bestimmte Zeit einwirken gelassen und wird anschließend mit vergleichsweise hohem Aufwand wieder entfernt. Insbesondere das Entfernen von Restmengen aus dem Behältnis und die Überwachung der vollständigen Entfernung ist aufwändig. Anschließend erfolgt die Übergabe der sterilisierten Behälter an ein Transportsystem, welches die Gebinde zu der aseptisch abgekapselten Abfüllvorrichtung transportiert. Um auch während des Transports die Sterilität der Gebinde aufrecht zu erhalten, sind besondere Schutzmaßnahmen erforderlich.

Die Problematik der Überwachung von Restmengen chemischer Sterilisationsmittel und der damit verbundene längere steril zu haltende Transportweg verteuern diese Verfahren sehr. Um dies zu vermeiden, sind aus dem Stand der Technik Verfahren bekannt, welche alternative Sterilisationsverfahren nutzen. Neuere Entwicklungen versuchen, die Mengen eingesetzter Chemikalien zu reduzieren und nutzen ionisierende Strahlung um eine Keimreduzierung zu erreichen. Der große Vorteil hierin besteht in der Verringerung oder Vermeidung des Einsatzes chemischer Stoffe. Die eingesetzte Strahlung besteht in den meisten Anwendungen aus beschleunigten Elektronen, die in einer entsprechenden Anlage erzeugt und auf den zu sterilisierenden Bereich des Behältnisses geleitet werden.

Wie beispielsweise aus der JP 2002-255125 oder der WO 99/39751 A ist eine Vorrichtung zum Sterilisieren von Behältnissen bekannt. Dabei ist eine außerhalb des Behältnisses vorgesehene Strahlungsquelle vorgesehen, die Strahlung auf das Behältnis richtet. Dabei muss insbesondere bei dem Gegenstand der WO 99/39751 A der Ladungsträgerstrahl für die Innensterilisation des Behältnisses die Behältniswand durchdringen. Die Behältnisse werden auf einem Förderband transportiert und an der Strahlungsquelle vorbeigeführt. Dies ist insbesondere für die Sterilisation des Bodenbereichs des Behältnisses nachteilig, da dieser für den Ladungsträgerstrahl nicht direkt zugänglich ist.

Die DE 198 82 252 T1 beschreibt eine Technik zur Innensterilisation eines Behälters mittels Elektronen. Dabei ist ebenfalls eine Elektronenstrahlquelle vorgesehen, die Strahlung von außerhalb in das Innere des Behältnisses richtet. Der Transport der Behältnisse erfolgt jedoch auch bei dem Gegenstand dieser Druckschrift über ein Transportband.

Aus der EP 198 29 21 A1 ist eine Vorrichtung zum Sterilisieren von Behältnissen bekannt, mittels welcher die Behältnisse vor deren Befüllung sterilisiert werden können. Speziell soll durch die in EP 198 29 21 A1 beschriebene Vorrichtung die Innenwandung von zu befüllenden Behältnissen, insbesondere von Flaschen, welche eine Mündung aufweisen, sterilisiert werden.

Die EP 2 123 580 A1 beschreibt eine Haltevorrichtung zum Greifen von Flaschen an deren Außenseite, welche einen besonders geringen Abschattungsbereich für Elektronenstrahlen bietet. Dazu werden von gegenüberliegenden Seiten der Flasche dünne Platten unter den Greifring geschoben.

Die DE 60 2006 000 257 T2 offenbart ebenfalls ein Haltesystem für Flaschen während deren Sterilisation, welches jeweils zwei Flaschen greift und so ausrichtet, dass die Flaschenlängsachsen auf einer gemeinsamen Achse liegen. Die Sterilisation erfolgt mittels Elektronenstrahlen.

Aus der WO 2008 081 868 ist eine Sterilisationsapparatur bekannt, welche die Menge entstehender Stickoxide während der Sterilisation von Behältnissen mittels Elektronenstrahlen reduziert.

Die EP 1 748 951 B1 und auch die US 2005 158 218 offenbaren jeweils ein Transportsystem mittels welchem Behältnisse in einer Behältnisbehandlungsanlage transportiert werden können. Die Anlage schließt auch eine Sterilisationseinrichtung ein, durch welche die Behältnisse mittels des Transportsystems transportiert werden.

Aus der EP 0 885 142 B1 ist ein Verfahren zur Innensterilisation von Fliesmittelpackungen bekannt. Bei diesem Verfahren werden ebenfalls beschleunigte Elektronen eingesetzt, jedoch sollen hohe Beschleunigungsspannungen vermieden werden, da als unerwünschter Nebeneffekt dieser hohen Beschleunigungsspannungen Röntgenstrahlen erzeugt werden und diese wiederum mit einem Bleimantel abgeschirmt werden müssten. Um gleichermaßen ein gleichmäßiges Sterilisationsergebnis zu erhalten, wird zusätzlich zum Elektronenstrahl ein Gasstrom eingesetzt, welcher mit dem Elektronenstrahl interagiert und die Ladungsträgerverteilung verbessert. Über den Transport der zu sterilisierenden Behältnisse wird in EP 0 885 142 B1 keine Angabe gemacht.

Die EP 2 279 952 A1 offenbart ein Verfahren zum Befüllen von Flaschen mit einer Flüssigkeit, wobei die Flasche zunächst blasgeformt, sterilisiert und danach befüllt wird. Die Sterilisation wird hierbei durch Wasserstoffperoxid durchgeführt.

Die aktuellen Systeme, die am Markt erhältlich sind und zur Sterilisation eingesetzt werden, bestehen demnach entweder aus einer Elektronenerzeugungsvorrichtung und einem Strahlfinger zur Entkeimung der inneren Oberflächen oder aber aus Elektronenerzeugungsvorrichtung die von außen durch den Behälter auch die inneren Oberflächen behandeln. Die Behandlung von Außen erfordert üblicherweise wesentlich stärker beschleunigte Elektronen, da die Wand des zu behandelnden Behälters durchdrungen werden muss und trotz der Schwächung die Elektronen immer noch genügend Energie enthalten müssen um an der inneren Oberfläche sterilisierend zu wirken. Die dabei entstehende Röntgenstrahlung muss durch geeignete Abschirmungen von der Umwelt abgeschirmt werden. Zusätzlich wird das gesamte Behältermaterial mit einer Strahlendosis belastet. Anordnungen die mit einem Strahlfinger arbeiten kommen in der Regel mit wesentlich geringeren Beschleunigungsspannungen und damit mit geringeren Abschirmungen und geringeren Strahlendosen im Behältermaterial aus. Um die Behälter auch außen zu Sterilisieren ist allerdings ein weiterer Prozessschritt not wendig. Diese Außenbehandlung kann vor, während oder aber auch nach der Innenbehandlung durchgeführt werden.

Die aus dem Stand der Technik bekannten Sterilisationsverfahren weisen daher den Nachteil auf, dass sie entweder mit chemischen Sterilisationsmitteln arbeiten oder bei einer Sterilisation mittels Ladungsträgern nicht die gesamte Oberfläche eines Behältnisses sterilisieren können. Insbesondere weist die innere und/oder die äußere Behälteroberfläche bei einer Sterilisierung mittels Ladungsträgerstrahlen Bereiche auf, welche durch Teile des Transportsystems überdeckt werden und so für die Ladungsträgerstrahlen nicht oder nur schlecht zugänglich sind. Die in diesen Bereichen auftreffende Strahlungsleistung reicht oft für eine ausreichende Sterilisierung nicht aus oder es müssen besonders starke Strahlenquellen eingesetzt werden, die aber die damit verbundenen Nachteile wie z.B. verstärktes Austreten von Röntgenstrahlung aufweisen.

Der vorliegenden Erfindung liegt daher die Aufgabe zugrunde, eine Vorrichtung und ein Verfahren zum Sterilisieren von Behältnissen und insbesondere einer Außenwandung von Behältnissen mittels beschleunigter Ladungsträger welche in einer Ladungsträgererquelle erzeugt und mit einer Beschleunigungseinrichtung in Richtung des Behältnisses beschleunigt werden bereitzustellen, wobei die Behältnisse mittels einer Transporteinrichtung, welche mindestens ein Halteelement zum Halten mindestens eines Behältnisses aufweist, gegenüber der Ladungsträgerquelle relativbewegt sind und dieses Halteelement nicht im Ladungsträgerstrahl angeordnet ist, um zu vermeiden, dass bei einer Außensterilisation des Behältnisses Ladungsträger von der Ladungsträgerquelle bzw. der Beschleunigungseinrichtung auf das Halteelement oder einen Abschnitt des Halteelements treffen und so ein Ladungsträgerstrahlschatten auf der Behältnisoberfläche entsteht.

Weiterhin ist eine Aufgabe der Erfindung, eine Anlage zum Behandeln von Behältnissen bereitzustellen, welche eine derartige Vorrichtung umfasst. Als Außenwandung wird in diesem Zusammenhang eine Außenoberfläche des Behältnisses verstanden, wobei eine Außenwandung auch ein Abschnitt bzw. Anteil oder Bereich der gesamten Außenoberfläche oder auch die gesamte Außenoberfläche des Behältnisses darstellen kann.

Dies wird erfindungsgemäß durch eine Vorrichtung nach Anspruch 1, eine Anlage nach Anspruch 10 und ein Verfahren nach Anspruch 14 erreicht. Vorteilhafte Ausführungsformen und Weiterbildungen sind Gegenstand der Unteransprüche.

In einer erfindungsgemäßen Vorrichtung zum Sterilisieren von Behältnissen und insbesondere einer Außenwandung von Behältnissen mittels beschleunigter Ladungsträger mit einer richtung, welche die Ladungsträger - insbesondere in Richtung einer Außenwandung des Behältnisses - (denkbar wäre jedoch auch eine Umlenkung der Ladungsträger nach deren Beschleunigung) beschleunigt, wobei die Behältnisse mittels einer Transporteinrichtung gegenüber der Ladungsträgerquelle entlang eines vorgegebenen Transportpfades relativ beweglich sind und die Transporteinrichtung eine Vielzahl von Halteelementen zum Halten mindestens eines Behältnisses aufweist, sind die Halteelemente zumindest teilweise in das Innere der Behältnisse einführbar und mit einer Innenwandung der Behältnisse kontaktierbar (bzw. in Kontakt bringbar), um zu gewährleisten, dass bei einer Außensterilisation des Behältnisses Ladungsträger von der Ladungsträgerquelle bzw. der Beschleunigungseinrichtung direkt auf eine Außenseite des Behältnisses geleitet werden können ohne dass das Halteelement oder mindestens ein Abschnitt des Halteelements zwischen der Ladungsträgerquelle bzw. der Beschleunigungseinrichtung und einer Außenseite des Behältnisses angeordnet ist. Erfindungsgemäß ist dabei ein in das Innere des Behältnisses einführbarer Anteil des Halteelementes in seiner Dimension zumindest in einer Richtung, welche senkrecht zur Längsrichtung des Behältnisses steht, veränderbar.

Insbesondere ist daher kein Abschnitt des Halteelements in einem Strahlengang der Elektronenstrahlung zwischen der Beschleunigungseinrichtung und dem Behältnis bzw. der Aussenoberfläche des Behältnisses angeordnet.

Demnach wird vorteilhaft der Prozess der Außenentkeimung vom Prozess der Innenentkeimung getrennt um eine Behandlung aller Behälteroberflächen ohne Einfluss von jeglicher Beschattung durch Behältertransportelemente zu ermöglichen. Dazu sind die Behältertransportelemente die die Behälter im Bereich der Außenentkeimung transportieren, mit einem Greifsystem ausgerüstet, welches die Behälter von innen greifen kann und so einer störungsfreien Bestrahlung der Außenfläche nicht entgegensteht. Nach der Außensterilisation erfolgt für die Sterilisation der Innenseiten des Behälters ein Umgreifen des Greifsystems, so dass das Behältnis von außen gegriffen wird und eine störungsfreie Bestrahlung der Innenfläche ermöglicht wird. Die Reihenfolge der dieser Innen- und Außensterilisation kann auch in umgekehrter Reihenfolge ablaufen.

Das Greifsystem ist dabei vorteilhaft so aufgebaut, dass es den hygienischen Anforderungen eines Sterilisationsprozesses genügt und bevorzugt selbst leicht zu sterilisieren ist. Insbesondere ist das Greifsystem so aufgebaut, dass durch die Halteelemente keine Verunreinigungen von einem Behältnis auf das nächste von diesem Halteelement gegriffene Behältnis übertragen werden. In einem Ausführungsbeispiel ist das Halteelement bzw. der Innengreifer übertragen werden. In einem Ausführungsbeispiel ist das Halteelement bzw. der Innengreifer eine Art Spannzange die ohne weitere Klemmvorrichtungen den Behälter sicher transportieren kann.

Das Greifsystem ist bevorzugt so aufgebaut, dass es den Behälter bereits an den Übergabestellen, insbesondere zwischen benachbarten Transportsystemen, innen greifen kann. Ein Umgreifen ist somit nicht nötig. Damit wird die Gefahr von Kontaminationen weiter reduziert. Für das Eingreifen in ein Behältnis ist bevorzugt vorgesehen, dass das Halteelement oder das Behältnis in der Lage ist eine Bewegung bzw. einen Hub in Richtung der Behälterachse auszuführen. Dieser Hub kann beispielsweise mittels eines Kurvensegmentes, einer umlaufenden Kurve, einer pneumatischen Betätigung, einer Servo-angetriebenen Linearführung oder mit allen anderen denkbaren Antrieben ausgeführt werden. Es ist sowohl denkbar, dass das Halteelement im wesentlichen entlang der Behältnislängsrichtung in Richtung des Behältnisses (und/oder weg von diesem) bewegt wird oder auch dass das Behältnis selbst in Richtung des Halteelements (und/oder von diesem weg) bewegt wird.

In einer bevorzugten Ausführungsform der Vorrichtung ist das Innere des Behältnisses zumindest im Bereich einer Mündung bzw. eines Gewindes durch das Halteelement kontaktierbar bzw. in Kontakt bringbar. Der Bereich des Behältnisverschlusses ist für den Transport mittels des innengreifenden Halteelements besonders bevorzugt, da in diesem Bereich üblicherweise eine Öffnung ist, durch die das Halteelement eingeführt werden kann. Außerdem erweitert sich im Bereich des Behältnisverschlusses in vielen Ausführungsformen, insbesondere bei Getränkeflaschen, das Behältnis, so dass einem Herabfallen des Behältnisses nach einer Veränderung der Geometrie des Halteelements, insbesondere einer Verbreiterung dessen Umfangs und/oder Durchmessers senkrecht zur Längsachse des Behältnisses, effektiv vorgebeugt wird. Insbesondere ist in einer bevorzugten Ausführungsform vorgesehen, dass das in seiner Geometrie veränderte Halteelement ohne Rückführung in die vorherige Geometrie nicht durch die Behältnisöffnung zurückgeführt werden kann.

Erfindungsgemäß ist ein in das Innere des Behältnisses einführbarer Anteil des Halteelementes in seiner Dimension zumindest in einer Richtung, welche senkrecht zur Längsrichtung des Behältnisses steht, veränderbar.

Bevorzugt ist dieser Anteil des Halteelementes bevorzugt eine Spannhülse. Es sind auch flexible, verformbare, aufblasbare oder ähnliche Ausführungen des Halteelements denkbar.

Weiterhin ist bevorzugt, das Halteelement drehbar ist, um ein von dem Halteelement erfasstes Behältnis entlang dessen Längsachse drehen zu können. Durch diese Ausführung ist es möglich, das Behältnis während der Bestrahlung durch den Ladungsträgerstrahl drehen zu können, so dass alle äußeren Oberflächen zumindest zeitweise der Strahlungsquelle zugewandt sind und somit direkt von dieser mit den Ladungsträgern beaufschlagt werden können. Bevorzugt erfolgt eine definierte Drehung um die Behältnislängsachse, so dass die Außenwandung des Behältnisses mit einer definierten Strahlendosis beaufschlagt wird. Insbesondere wird so gewährleistet, dass eine gleichmäßige Bestrahlung der Außenwandung des Behältnisses erfolgt. Es ist jedoch auch denkbar, dass besonders für Verschmutzungen gefährdete Bereiche wie z.B. Rillen, Fugen, Schweißnähte, Oberflächenunebenheiten o.ä. mit einer höheren Strahlendosis beaufschlagt werden. Dies kann beispielsweise über nicht gleichförmige Rotationsgeschwindigkeiten erreicht werden.

Erfindungsgemäß ist ein in das Innere des Behältnisses einführbarer Anteil des Halteelementes in seiner Dimension zumindest in einer Richtung, welche senkrecht zur Längsrichtung des Behältnisses steht, veränderbar. Hierunter wird insbesondere verstanden, dass dieser Bereich des Halteelements hinsichtlich seiner radialen Richtung veränderbar ist, sich also beispielsweise in Richtung der Innenwandung des Behältnisses ausdehnen kann. Zu diesem Zweck kann das Halteelement beispielsweise Spreizelemente aufweisen.

Bei einer weiteren vorteilhaften Ausführungsform ist das in die Mündung der Behältnisse einführbare Halteelement eine Spannhülse oder weist eine Spannhülse auf. Diese Spannhülse kann in die Mündung der Behältnisse eingeführt werden und sich anschließend ausdehnen, um die Behältnisse an ihrer Innenwandung zu greifen.

Dabei ist diese Spannhülse vorteilhaft derart gestaltet, dass eine federnde Wirkung zum Greifen der Behältnisse erzeugt wird und vorteilhaft diese federnde Wirkung wenigstens überwiegend und bevorzugt allein durch die Ausführung und/oder Materialwahl der Spannhülse erfolgt bzw. erzeugt wird, derart, dass für die federnde Wirkung zum Greifen und Festhalten des Behältnisses keine weiteren Bauteile erforderlich sind.

Um eine möglichst hohe Effektivität zu erreichen und eine große Anzahl von Behältnissen in kurzen Zeitintervallen an der Strahlungsquelle vorbeiführen zu können, ist vorgesehen, dass eine Vielzahl von Halteelementen auf einem beweglichen Träger angeordnet ist, wobei der bewegliche Träger bevorzugt ein Transportrad oder Transportstern ist. Bevorzugt sind die Halteelemente mit diesem in einer im Wesentlichen ringförmigen Anordnung verbunden. Eine derartige Ausführungsform ist in den meisten Fällen kompatibel zu bekannten Transportanlagen, wie sie zur Behandlung von Behältnissen bereits eingesetzt werden. Somit wird ein hoher Durchsatz und evtl. sogar die Kompatibilität zu bestehenden Anlagen ermöglicht.

In einigen Fällen kann es vorkommen, dass die Transportgeschwindigkeit der Behältnisse so groß ist, dass die Verweilzeit der Behältnisse im Bereich des Ladungsträgerstrahls nicht für eine vollständige Sterilisierung ausreicht. Um die Arbeitsgeschwindigkeit des Transportsystems, z.B. des Transportrades oder Transportsterns nicht reduzieren zu müssen, ist in einer bevorzugten Ausführungsform vorgesehen, dass ein Abstand zwischen zwei benachbarten Halteelementen in einer Transportrichtung der Behältnisse veränderbar ist. Dies kann beispielsweise dadurch erreicht werden, dass der Abstand zwischen zwei benachbarten Halteelementen bevorzugt in dem Abschnitt eines Transportpfades, in welchem dieser den geringsten Abstand zu der Ladungsträgerquelle bzw. der Beschleunigungseinrichtung aufweist, reduziert ist.

Bevorzugt wird dies dadurch ermöglicht, dass ein Halteelement ein einem Arm angeordnet ist, welcher im Wesentlichen senkrecht zum Transportpfad ausgerichtet ist. Im Fall eines Transportrades verläuft ein solcher Arm demnach weitgehend in radialer Richtung. Bei einer beweglichen Lagerung oder Ausbildung eines solchen Arms ist es möglich den Arm derart entlang des Transportpfades zu bewegen, dass die Relativgeschwindigkeit zwischen dem von dem Haltelement des Arms getragenen Behältnisses und der Ladungsträgerquelle verändert wird. So ist insbesondere vorgesehen, den Arm bei Annäherung des Behältnisses an die Ladungsträgerquelle in Transportrichtung nach vorne zu schwenken, so dass die Relativgeschwindigkeit erhöht wird. Dadurch kommt das zu sterilisierende Behältnis früher in den Einflussbereich des Ladungsträgerstrahls. Überschreitet das Behältnis auf dem Transportpfad den Punkt der größten Annäherung zwischen Behältnis und Ladungsträgerquelle, ist es möglich, den Arm in Transportrichtung nach hinten zu schwenken, wodurch die Relativgeschwindigkeit zwischen dem von dem Haltelement des Arms getragenen Behältnisses und der Ladungsträgerquelle verringert wird.

Das Behältnis verbleibt somit länger im Einflussbereich des Ladungsträgerstrahls. In dem Abschnitt des Transportpfades, in welchem die Behältnisse den geringsten Abstand zu der Ladungsträgerquelle bzw. der Beschleunigungseinrichtung aufweisen, wirkt sich dies darin aus, dass die Halteelemente und damit auch die Behältnisse näher zusammenrücken und demnach entlang des Transportpfades eine geringeren Teilungsabstand aufweisen. Der Abstand zwischen zwei benachbarten Behältnissen entspricht bei vielen Ausführungsformen dem Anteil an beschleunigter Strahlung, welcher ungenutzt zwischen zwei zu sterilisierenden Behältnissen hindurch in die Umgebung abgegeben wird. Durch die oben beschriebene Ausführungsform kann daher auch eine Steigerung der Ausnutzung der beschleunigten Elektronen und damit der Energieausbeute erreicht werden.

Vorteilhaft wird die Relativgeschwindigkeit zwischen den Behältnissen und der Strahlungsquelle in dem Bereich des Transportpfades in dem die Behältnisse mit Strahlung beaufschlagt werden verändert, bevorzugt verringert. Um die Zeit der Strahlenbeaufschlagung für jeden Bereich der Außenwandung der Behältnisse bei gleichzeitig hoher Relativgeschwindigkeit zwischen den Behältnissen und der Strahlungsquelle zu verlängern, ist es möglich, mehrere Strahlungsquellen zu verwenden, welche jeweils andere Bereiche der Außenwandung des Behältnisses mit Strahlung beaufschlagen. Dadurch ist es auch möglich, die Rotationsgeschwindigkeit des Behältnisses um dessen Längsachse im Bereich der Strahlungsquellen zu reduzieren, da jede der Strahlungsquellen nur einen kleineren Bereich der Behältnisaußenwandung mit Strahlung beaufschlagen muss.

Um die Sterilität der mit den Ladungsträgern beaufschlagten Behältnisse längerfristig aufrecht erhalten zu können, umfasst die Vorrichtung bevorzugt einen Reinraum, welcher zumindest ein Abschnitt des eines Transportpfades der Behältnisse und bevorzugt auch der Halteelemente einschließt.

Vorzugsweise ist das Halteelement einer Vorrichtung zum Sterilisieren von Behältnissen in einer Richtung bewegbar, welche im Wesentlichen parallel zur Längsachse der Behältnisse verläuft. Dadurch wird es ermöglicht, dass die Halteeinrichtung in das Behältnisinnere eingeführt werden kann. Bei Behältnissen, welche eine Geometrie aufweisen, in welcher eine Öffnung zu Einführen der Halteeinrichtung nicht entlang der Längsachse der Behältnisse angeordnet ist, sind auch andere Bewegungsrichtungen des Halteelements zu dessen Einführen in das Behältnisinnere möglich. Weiterhin ist es möglich, dass die Halteeinrichtung nicht aktiv in das Innere des Behältnisses eingeführt wird, sondern dass das Behältnis in Richtung der Halteeinrichtung bewegt wird und somit das Behältnis über die Halteeinrichtung gestülpt wird. Auch eine Kombination beider Bewegungsformen ist möglich. Dies ermöglicht auch kompliziertere Bewegungspfade der Relativbewegung zwischen Behältnis und Halteeinrichtung. Je nach geometrischer Ausführung des Behältnisses, z.B. des Flaschenhalses kann dies vorteilhaft oder sogar notwendig sein.

Weiterhin ist eine Anlage zum Behandeln von Behältnissen bevorzugt, welche mindestens eine Vorrichtung zum Sterilisieren von Behältnissen umfasst wie sie oben beschrieben ist. Eine derartige Anlage ist dazu geeignet, Behältnisse an deren Außenseite vollständig, effizient und schnell mittels eines Ladungsträgerstrahls zu sterilisieren. Die Außenwandung des Behältnisses kann vollständig dem Ladungsträgerstrahl ausgesetzt werden und keinerlei Halteelemente werfen einen Ladungsträgerschatten auf die Behältnisoberfläche. Bevorzugt umfasst eine derartige Anlage eine weitere Vorrichtung zum Sterilisieren einer Innenwandung der Behältnisse. Somit kann eine vollständige Sterilisierung des Behältnisses ermöglicht werden.

Dabei ist es möglich, dass die Vorrichtung zum Sterilisieren der Innenwandung der Behältnisse eine Bestrahlungseinrichtung aufweist, welche die Innenwandung der Behältnisse mit Strahlung und insbesondere mit Ladungsträgern beaufschlagt. Vorteilhaft ist dabei diese Bestrahlungseinrichtung wenigstens abschnittsweise durch eine Mündung des Behältnisses in das Behältnis einführbar. Zu diesem Zweck kann die Vorrichtung eine Hubeinrichtung aufweisen, welche eine Relativbewegung zwischen den Behältnissen und der Bestrahlungseinrichtung in der Längsrichtung der Behältnisse erzeugt.

Bevorzugt ist daher eine Anlage, deren weitere Vorrichtung zum Sterilisieren mindestens ein Halteelement umfasst, mit welchem ein Behältnis an dessen Außenseite kontaktierbar ist und mittels welcher bevorzugt die Innenwandung des Behältnisses mittels einer zumindest abschnittsweise in das Behältnisinnere eingeführten Quelle für beschleunigte Ladungsträger mit Ladungsträgern beaufschlagbar ist. Durch diese Kombination mindestens zweier Sterilisationseinrichtungen, welche beide mit beschleunigten Ladungsträgern als Sterilisationsmedium arbeiten, ist es möglich, den Anteil chemischer Sterilisationsmittel weiter zu reduzieren oder sogar ganz auf chemische Desinfektionsmittel zu verzichten. Dies ist einerseits insbesondere im Hinblick auf die Kosten des Desinfektionsmittels und andererseits auf die Arbeitssicherheit im Umgang mit diesen Chemikalien und die Entsorgungskosten vorteilhaft.

Weiterhin ist eine Ausführungsform der Anlage bevorzugt, in welcher die Anlage mindestens ein Transportelement, bevorzugt einen Transportstern umfasst, welcher dazu geeignet ist, ein Behältnis von einer Vorrichtung zum Sterilisieren von Behältnissen zu übernehmen und an die weitere Vorrichtung zum Sterilisieren zu übergeben, wobei die Übergabe bevorzugt in einem Reinraum erfolgt. Dabei ist es möglich, die Vorrichtungen zum Sterilisieren in beliebiger Reihenfolge entlang des Transportpfades anzuordnen. Es sind demnach sowohl Ausführungsformen möglich, bei denen zunächst die Innensterilisation und anschließend die Außensterilisation des Behältnisses erfolgt als auch Ausführungsformen, bei denen zunächst die Außensterilisation und anschließend die Innensterilisation des Behältnisses erfolgt.

Die Ausgestaltung des/der für die Übergabe von einer Vorrichtung zum Sterilisieren an die folgende Vorrichtung zum Sterilisieren verwendeten Transportelementes/e kann dementsprechend angepasst werden. Insbesondere ist dabei vorgesehen, dass die in der entlang des Transportpfades ersten Vorrichtung zum Sterilisieren sterilisierte Fläche nicht durch das Transportelement mit Verunreinigungen kontaminiert wird. Bevorzugt ist das Transportelement daher steril bzw. weist sterile Kontaktflächen auf, mit denen das Behältnis kontaktiert wird. Bevorzugt befindet sich zumindest das Halteelement des Transportelements in einem Reinraum, so dass die Übergabe - insbesondere der gesamte Übergabeprozess von der entlang des Transportpfades ersten Vorrichtung zum Sterilisieren an die entlang des Transportpfades folgende Vorrichtung zum Sterilisieren - bevorzugt in einem Reinraum erfolgt. Es erfolgt vor dem Verwenden der Vorrichtung bevorzugt ein Sterilisieren des Reinraumes und/oder der Transportelemente, insbesondere mit einem chemischen Sterilisierungsmittel.

Bevorzugt weist die Anlage eine Einrichtung zum Befüllen von Behältnissen auf, wobei diese Einrichtung bevorzugt stromabwärts bezüglich einer Vorrichtung zum Sterilisieren angeordnet ist. Die sterilisierten Behältnisse werden bevorzugt über den gesamten Transportprozess zwischen der Vorrichtung zum Sterilisieren und der Einrichtung zum Befüllen steril gehalten. Daher befindet sich der Transportpfad für die Behältnisse bevorzugt in einem Reinraum.

Weiterhin ist ein Verfahren zum Sterilisieren von Behältnissen mittels beschleunigter Ladungsträger, welche in einer Ladungsträgererquelle erzeugt und mit einer Beschleunigungseinrichtung in Richtung des Behältnisses beschleunigt werden Gegenstand der Erfindung, wobei die Behältnisse mittels einer Transporteinrichtung, welche eine Vielzahl von Halteelementen zum Halten der Behältnisse aufweist, gegenüber der Ladungsträgerquelle relativbewegt werden, und die Halteelemente zumindest teilweise in das Innere der Behältnisses eingreifen um zu gewährleisten, dass bei einer Außensterilisation des Behältnisses Ladungsträger von der Ladungsträgerquelle bzw. der Beschleunigungseinrichtung direkt auf eine Außenseite des Behältnisses geleitet werden können ohne dass das Halteelement oder mindestens ein Abschnitt des Halteelements zwischen der Ladungsträgerquelle bzw. der Beschleunigungseinrichtung und einer Außenseite des Behältnisses angeordnet ist.

Weitere Vorteile und Ausführungsformen ergeben sich aus den beigefügten Zeichnungen:

### Darin zeigen:

- Fig. 1: eine schematische Darstellung einer Aufsicht auf eine Vorrichtung zum Sterilisieren von Behältnissen mittels beschleunigter Ladungsträger mit einem Ein- und Auslaufstern;
- Fig. 2: eine schematische Darstellung einer Seitenansicht einer Vorrichtung zum Sterilisieren von Behältnissen mittels beschleunigter Ladungsträger;
- Fig. 3: eine schematische Darstellung einer Aufsicht auf eine Vorrichtung zum Sterilisieren von Behältnissen mittels beschleunigter Ladungsträger mit einem Ein- und Auslaufstern analog Fig. 1, jedoch mit einer Möglichkeit zur Veränderung des Teilungsverzugs zwischen benachbarten Behältnissen zumindest im Bereich der Strahlungsquelle; und
- Fig. 4a - 4c: drei Darstellungen für ein Halteelement zum Halten der Behältnisse.

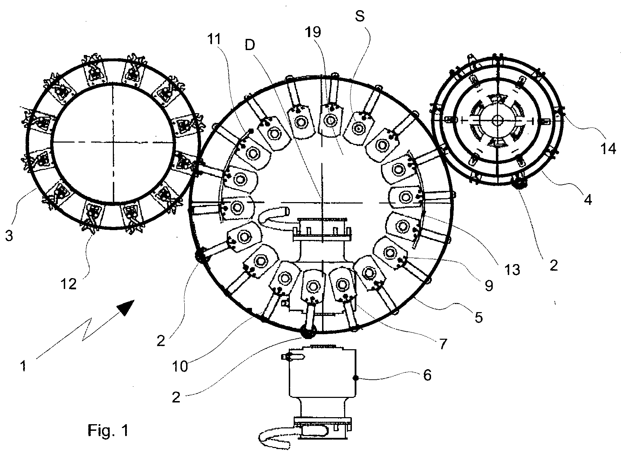

Figur 1 zeigt eine schematische Darstellung einer Aufsicht auf eine Vorrichtung 1 zum Sterilisieren von Behältnissen 2 mittels beschleunigter Ladungsträger mit einem Ein- 3 und Auslaufstern 4. Die Behältnisse 2 werden entlang eines Transportpfades 5, welcher sich mäanderförmig entlang der Vorrichtung erstreckt, an der Ladungsträgerquelle 6 vorbeigeführt. Dort werden die Behältnisse 2 mit Ladungsträgern beaufschlagt. Um die Effizienz und die Anzahl der pro Zeiteinheit sterilisierbaren Behältnisse 2 zu erhöhen, ist es wie hier gezeigt möglich, dass eine (oder mehrere, hier nicht gezeigte) weitere Ladungsträgerquelle(n) 7 entlang des Transportpfades 5 angeordnet sind, welche andere Bereiche des Behältnisses mit Ladungsträgern beaufschlagen. Auch können eine oder mehrere Bestrahlungseinrichtungen vorgesehen sein, welche einen Boden der Behältnisse von außen bestrahlen.

Die Behältnisse 2 werden von einem (hier nicht im Detail gezeigten Halteelement) gehalten, welches Teil eines Haltesystems 9 ist. Dieses Haltesystem 9 weist weiterhin einen Arm bzw. Ausleger 10 auf, an welchem das Halteelement angeordnet ist, an dem wiederum das Behältnis hängen kann. Zum Einführen des Halteelements in das jeweilige Behältnis 2 ist eine erste Steuerkurve 11 im Bereich des Einlaufsterns 3 vorgesehen, mittels welchem der Arm 10 derart geführt werden kann, dass das Halteelement in das Behältnisinnere eingeführt werden kann. Nach dem Einführen des Halteelements in das Behältnisinnere kann dieses das jeweilige Behältnis 2 von dem Greifelement 12 des Einlaufsterns übernehmen und weiter entlang des Transportpfades 5 transportieren. Eine weitere Steuerkurve 13 ist im Bereich der Übergabe der sterilisierten Behältnisse 2 an den Auslaufstern 4 bzw. dessen Greifelemente 14 vorgesehen. Anstelle oder neben Steuerkurven könnten auch separate (z.B. pneumatische, hydraulische oder elektromotorische) Antriebe vorgesehen sein, welche die Halteelemente antreiben.

Das Bezugszeichen 19 bezieht sich auf einen um eine Drehachse D drehbaren Träger bzw. Transportstern, an dem die einzelnen Halteelemente bzw. die Arme 10, bevorzugt schwenkbar bezüglich Schwenkachsen S gegenüber dem Träger 19 angeordnet sind.

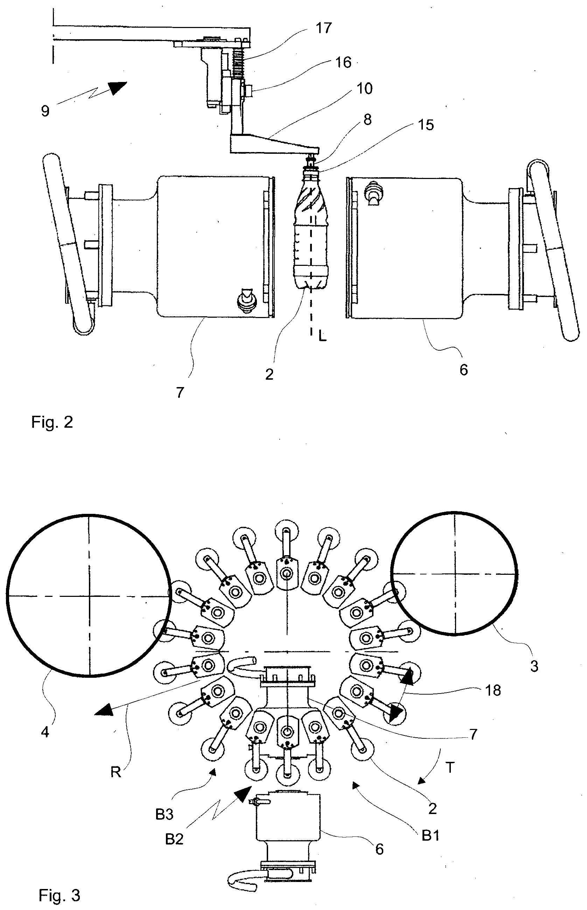

In Figur 2 ist eine schematische Darstellung einer Seitenansicht einer Vorrichtung zum Sterilisieren von Behältnissen mittels beschleunigter Ladungsträger gezeigt. In dieser Darstellung ist ein Behältnis 2 gezeigt, welches im Bereich dessen Verschlusses bzw. Öffnung 15 von dem Halteelement 8 gehalten wird. Das Halteelement 8 ist nur teilweise zu sehen, da der in das Behältnisinnere hineinragende Anteil durch das Behältnis 2 verdeckt ist.

Seitlich des Behältnisses 2 sind in diesem Ausführungsbeispiel zwei Ladungsträgerquellen 6 und 7 gezeigt, mittels welcher die Außenwandungen des Behältnisses 2 mit Ladungsträgern beaufschlagt werden können. In dieser Darstellung ist auch der Arm 10 des Haltesystems 9 gut zu erkennen, an welchem das Halteelement 8 angeordnet ist. Das Haltesystem 9 weist weiterhin eine Rolle 16 auf, welche auf einer der Steuerkurven 11 oder 13 entlangrollen kann und das Haltesystem 9 entgegen der Kraft einer Feder 17, insbesondere mittels einer Führungseinrichtung auf- und abwärts bewegen kann.

Fig. 3 zeigt eine schematische Darstellung einer Aufsicht auf eine Vorrichtung 1 zum Sterilisieren von Behältnissen 2 mittels beschleunigter Ladungsträger mit einem Ein- 3 und Auslaufstern 4 analog Fig. 1, jedoch mit einer Möglichkeit zur Veränderung der Teilung 18 (bzw. zum Erreichen eines Teilungsverzugs) zwischen benachbarten Behältnissen 2 zumindest im Bereich B2 der Strahlungsquelle. Wie auch bei der in Fig. 1 gezeigten Ausführungsform werden die Behältnisse 2 entlang eines Transportpfades 5 an der/den Ladungsträgerquelle/n 6 und 7 vorbeigeführt.

Um bei vorgegebener Rotationsgeschwindigkeit des Transportsterns 19 die Verweilzeit der Behältnisse 2 im Bereich B2 der Strahlungsquelle/n zu verlängern, ist es möglich, die Teilung 18 zwischen benachbarten Behältnissen 2 zu verändern, also einen Teilungsverzug zu erzeugen. Insbesondere ist es möglich, den Arm bzw. Ausleger 10 des Haltesystems 9 im Bereich B1 vor der Strahlungsquelle entlang des Transportpfades 5 vorwärts (demnach in Transportrichtung T) zu schwenken. Dadurch wird die Teilung 18 zum vorherigen Behältnis 2 verringert. Somit folgen die Behältnisse 2 im Bereich B2 der Strahlungsquelle/n dichter aufeinander und weniger Strahlung geht ungenutzt zwischen benachbarten Behältnissen 2 hindurch.

Während der Beaufschlagung der Behältnisse 2 mit Ladungsträgern wird der Arm 10 des jeweiligen Haltesystems 9 wieder in die Ausgangsposition und bevorzugt sogar über diese Ausgangsposition hinaus zurück geschwenkt, so dass während der Bestrahlung die gezeigte verringerte Teilung vorliegt.

Damit werden die Behältnisse während der Bestrahlung d.h. in dem Bereich B2, in dem sie von Ladungsträgern erreichbar sind, verzögert, so dass deren Geschwindigkeit gegenüber den Strahlungsquellen 6, 7 verlangsamt ist. Damit ist die resultierende Umfangsgeschwindigkeit der Halteelemente 8 im Bereich der Bestrahlungseinrichtungen geringer als in dem Bewegungsbereich außerhalb der Bestrahlungseinrichtungen 6, 7.

Durch die Änderung der Teilung bzw. durch den Teilungsverzug kann ggfs. sogar mit nur einer Bestrahlungseinrichtung ausgekommen werden, insbesondere, wenn sich die Behältnisse 2 zusätzlich um ihre Längsachse L (vgl. Fig.2) drehen.

Durch das oben beschriebene Zurückschwenken wird erreicht, dass der Arm 10 wieder weitgehend in radialer Richtung R nach außen verläuft (Bereich B3). Die Teilung 18 wird somit wieder auf den ursprünglichen Wert verändert und die Übergabe der sterilisierten Behältnisse 2 an den Auslaufstern 4 bzw. dessen Greifelemente 14 kann erfolgen.

Die Behältnisse 2 werden damit bevorzugt während ihrer Bewegung entlang ihres Transportpfades d.h. insbesondere zwischen der Übernahme von dem Einlaufstern und der Übergabe an den Auslaufstern wenigstens einmal beschleunigt und/oder einmal verzögert (insbesondere gegenüber einer Transportgeschwindigkeit, welche sich aus der Drehung des Trägers 19 ergibt).

Wie oben erwähnt, kann sich der gesamte Transportpfad der Behältnisse auch innerhalb eines (nicht gezeigten) Reinraums befinden. Dieser Reinraum kann dabei auch bereits vor dem Einlaufstern beginnen und sich auch über den Auslaufstern 4 hinaus erstrecken.

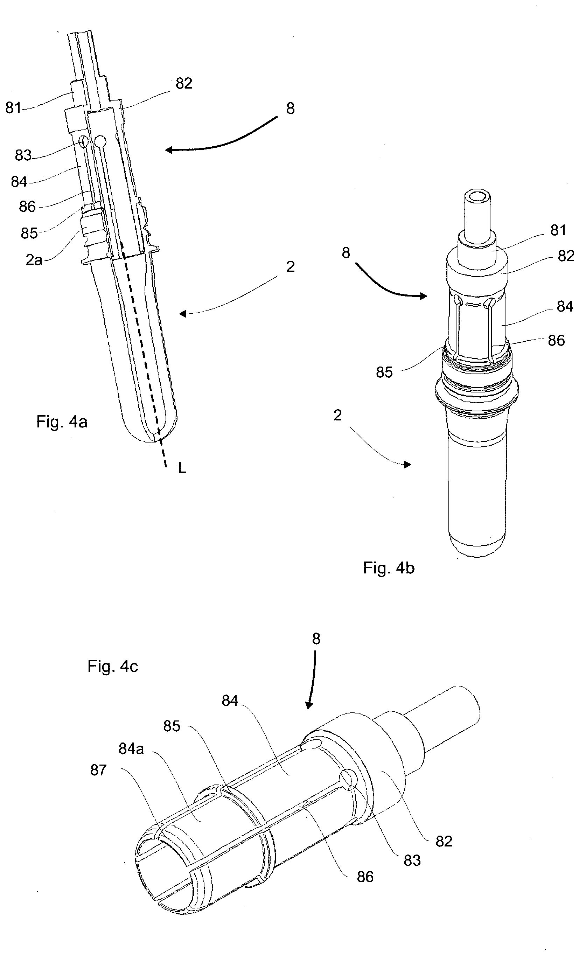

Die Fig. 4a - 4c zeigen drei Darstellungen eines - hier als Spannhülse ausgeführten - Halteelements 8 zum Halten der Behältnisse. Dieses Halteelement 8 ist dabei als passives Halteelement ausgeführt, welches das Behältnis bzw. den Vorformling 2 nur durch eine Federkraft von Haltestegen hält.

Im Einzelnen weist das Halteelement 8 einen Basisabschnitt 82, auf, der hier ringförmig ausgebildet ist. An diesem Basisabschnitt sind mehrere Haltestege bzw. Greifabschnitte 84 angeordnet, die sich in einer Längsrichtung L des Behältnisses 2 erstrecken. Dabei können Endabschnitte 84a dieser Greifabschnitte (vgl. Fig. 4c) in das Behältnis 2 eindringen. Weiterhin ist an den einzelnen Greifabschnitten 84 vorteilhaft ein radial nach außen ragender Vorsprung 85 angeordnet, der ein weiteres Eindringen der Greifabschnitte 84 und damit des Halteelements 8 in das Behältnis verhindert. Auf diese Weise kann in relativ genauer Weise die Kraft, die für ein späteres Abziehen der Behältnisse 2 von den Haltelementen nötig ist, festgelegt werden. Genauer stoßen die einzelnen Vorsprünge an einer Mündung 2a des Behältnisses 2 an. Die Greifabschnitte 84 weisen vorteilhaft auch einen kreisringsegmentförmigen Querschnitt auf.

Bei der hier gezeigten Ausführungsform weist das Halteelement 8 insgesamt fünf dieser Greifabschnitte 84 auf. Es könnten jedoch auch mehr oder weniger dieser Greifabschnittte vorgesehen sein.

Zwischen den einzelnen Greifabschnitten 84 sind jeweils Spalte 86 ausgebildet, welche ein Zusammendrücken der Greifabschnitte 84 ermöglichen. Die einzelnen Greifabschnitte 84 sind daher elastisch gegenüber ihrem Basisabschnitt 82 angeordnet. Der Basisabschnitt ist wiederum an einem Träger 81 angeordnet. Die einzelnen Spalte 86 münden hier bevorzugt in in etwa kreisförmig ausgebildete Löcher 83.

Vorteilhaft ist das Halteelement 8 einteilig ausgebildet und insbesondere sind die Greifabschnitte 84 mit dem Basisabschnitt 82 einteilig ausgebildet. Das Bezugszeichen 87 kennzeichnet am unteren Ende der einzelnen Greifabschnitte ausgebildete Einführschrägen, um auf diese Weise das Halteelement in das Behältnis einführen zu können. Bei einem Einführen des Halteelements in das Behältnis werden die einzelnen Greifabschnitte 84 zusammengedrückt und entwickeln so eine Gegenkraft, die gegen die Innenwandung der Mündung des Behältnisses 2 wirkt.

Es wird darauf hingewiesen, dass das hier beschriebene Halteelement auch allgemein zum Halten von Behältnissen verwendet werden kann und daher insbesondere nicht nur auf die hier beschriebene Vorrichtung zum Sterilisieren von Behältnissen gerichtet ist. Insbesondere kann das hier gezeigte Halteelement auch zum Halten anderer Behältnisse, wie insbesondere von Kunststoffflaschen (aber ggfs. auch zum Halten - insbesondere kleinerer - Glasflaschen) verwendet werden. Die Anmelderin behält sich daher vor, ggfs. im Rahmen von Teilanmeldungen Schutz für das hier beschriebene Halteelement zu beanspruchen, wobei die hier erläuterten Merkmale auch in beliebiger Weise kombinierbar sind.

Insbesondere weist das Halteelement bevorzugt ein Basiselement und eine Vielzahl von derart elastisch ausgebildeten und/oder elastisch an diesem Basiselement angeordneten und einteilig mit diesem Basiselement ausgebildeten Greifabschnitten auf, die in das Behältnis einführbar sind.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Vorrichtung zum Sterilisieren von Behältnissen
- 2: Behältnis
- 2a: Mündung des Behältnisses
- 3: Einlaufstern
- 4: Auslaufstern
- 5: Transportpfad
- 6: Erste Ladungsträgerquelle
- 7: Weitere Ladungsträgerquelle
- 8: Halteelement
- 9: Haltesystem
- 10: Arm, Ausleger
- 11: Erste Steuerkurve
- 12: Greifelement des Einlaufsterns
- 13: Weitere Steuerkurve
- 14: Greifelement des Auslaufsterns
- 15: Öffnung
- 16: Rolle
- 17: Feder
- 18: Teilung
- 19: Träger, Transportstern
- 81: Träger
- 82: Basisabschnit
- 83: Loch
- 84: Greifabschnit
- 84a: Endabschnitt des Greifabschnitts
- 85: Vorsprung
- 86: Spalt
- 87: Einführschräge
- R: radiale Richtung
- D: Drehachse
- S: Schwenkachse
- T: Transportrichtung
- L: Längsrichtung der Behältnisse
- B1-B3: Bereiche

## Patentansprüche

1. Vorrichtung (1) zum Sterilisieren von Behältnissen (2) und insbesondere einer Außenwandung von Behältnissen (2) mittels beschleunigter Ladungsträger mit einer Strahlungsquelle (6, 7). nämlich einer Ladungsträgerquelle (6, 7) zum Erzeugen der Ladungsträger, und mit einer Beschleunigungseinrichtung, welche die Ladungsträger
- insbesondere in Richtung einer Außenwandung der Behältnisse - beschleunigt,
wobei die Behältnisse (2) mittels einer Transporteinrichtung (19) gegenüber der Ladungsträgerquelle (6, 7) entlang eines vorgegebenen Transportpfades (5) relativbeweglich sind und die Transporteinrichtung (19)
eine Vielzahl von auf einem beweglichen Träger angeordneten Halteelementen (8) zum Halten mindestens eines Behältnisses (2) aufweist, um die Behältnisse (2) an der Strahlungsquelle (6, 7) vorbeizuführen,
**dadurch gekennzeichnet, dass**
die Halteelemente (8) zumindest teilweise in das Innere ders Behältnisses (2) einführbar sind und mit einer Innenwandung ders Behältnisses (2) in Kontakt bringbar sind und ein in das Innere des Behältnisses (2) einführbarer Anteil des Halteelementes (8) in seiner Dimension zumindest in einer Richtung, welche senkrecht zur Längsrichtung des Behältnisses steht, veränderbar ist,

2. Vorrichtung (1) zum Sterilisieren von Behältnissen (2) nach Anspruch 1,
**dadurch gekennzeichnet, dass**
das Innere des Behältnisses (2) zumindest im Bereich einer Mündung (15) durch das Halteelement (8) kontaktierbar ist.

3. Vorrichtung (1) zum Sterilisieren von Behältnissen (2) nach Anspruch 1 oder 2,
**dadurch gekennzeichnet, dass**
das Halteelement (8) drehbar ist, um ein von dem Halteelement (8) erfasstes Behältnis (2) bezüglich dessen Längsachse (L) drehen zu können.

4. Vorrichtung (1) zum Sterilisieren von Behältnissen (2) nach wenigstens einem der vorangegangenen Ansprüche,
**dadurch gekennzeichnet, dass**
das in die Mündung der Behältnisse einführbare Halteelement (8) eine Spannhülse ist.

5. Vorrichtung (1) nach Anspruch 4,
**dadurch gekennzeichnet, dass**
die Spannhülse so gestaltet ist, dass die federnde Wirkung zum Greifen der Behältnisse allein durch die Ausführung und/oder Materialwahl der Spannhülse erfolgt, derart, dass für die federnde Wirkung zum Greifen und Festhalten des Behältnisses keine weiteren Bauteile erforderlich sind.

6. Vorrichtung (1) zum Sterilisieren von Behältnissen (2) nach wenigstens einem der vorangegangenen Ansprüche,
**dadurch gekennzeichnet, dass**,
der bewegliche Träger (19) ein Transportrad oder Transportstern ist.

7. Vorrichtung (1) zum Sterilisieren von Behältnissen (2) nach wenigstens einem der vorangegangenen Ansprüche,
**dadurch gekennzeichnet, dass**
ein Abstand zwischen zwei benachbarten Halteelementen (8) in einer Transportrichtung (T) der Behältnisse veränderbar ist.

8. Vorrichtung (1) zum Sterilisieren von Behältnissen (2) nach wenigstens einem der vorangegangenen Ansprüche,
**dadurch gekennzeichnet, dass**
die Vorrichtung (1) einen Reinraum umfasst, welcher zumindest einen Abschnitt eines Transportpfades (5) der Behältnisse (2) und bevorzugt auch der Halteelemente (8) einschließt.

9. Vorrichtung (1) zum Sterilisieren von Behältnissen (2) nach wenigstens einem der vorangegangenen Ansprüche,
**dadurch gekennzeichnet, dass**
das Halteelement (8) in einer Richtung bewegbar ist, welche im Wesentlichen parallel zur Längsachse der Behältnisse (2) verläuft.

10. Anlage zum Behandeln von Behältnissen (2), welche mindestens eine Vorrichtung (1) zum Sterilisieren von Behältnissen (2) nach wenigstens einem der vorangegangenen Ansprüche aufweist,
**dadurch gekennzeichnet, dass**
die Anlage eine weitere Vorrichtung zum Sterilisieren einer Innenwandung der Behältnisse aufweist.

11. Anlage zum Behandeln von Behältnissen (2) nach Anspruch 10
**dadurch gekennzeichnet, dass**
die weitere Vorrichtung zum Sterilisieren mindestens ein Halteelement umfasst, mit welchem ein Behältnis (2) an dessen Außenseite in Kontakt bringbar ist und mittels welcher bevorzugt die Innenwandung des Behältnisses (2) mittels einer zumindest abschnittsweise in das Behältnisinnere eingeführten Quelle für beschleunigte Ladungsträger mit Ladungsträgern beaufschlagbar ist.

12. Anlage zum Behandeln von Behältnissen (2) nach Anspruch 10,
**dadurch gekennzeichnet, dass**
die Anlage mindestens ein Transportelement (19), bevorzugt einen Transportstern umfasst, welcher dazu geeignet ist, ein Behältnis (2) von einer Vorrichtung zum Sterilisieren von Behältnissen (2) zu übernehmen und an eine weitere Vorrichtung zum Sterilisieren zu übergeben.

13. Anlage zum Behandeln von Behältnissen (2) nach einem der Ansprüche 10-12,
**dadurch gekennzeichnet, dass**
die Anlage eine Einrichtung zum Befüllen von Behältnissen (2) aufweist und diese Einrichtung stromabwärts bezüglich der Vorrichtung (1) zum Sterilisieren angeordnet ist.

14. Verfahren zum Sterilisieren von Behältnissen (2) mittels beschleunigter Ladungsträger welche in einer Strahlunasauelle (6. 7), nämlich einer Ladungsträgerquelle (6, 7), erzeugt und mit einer Beschleunigungseinrichtung in Richtung des Behältnisses (2) beschleunigt werden, wobei die Behältnisse (2) mittels einer Transporteinrichtung (19), welche eine Vielzahl von Halteelementen (8) zum Halten der Behältnisses (2) aufweist, gegenüber der Ladungsträgerquelle (6, 7) relativ bewegt werden, wobei vorgesehen ist, dass die Vielzahl von Halteelementen (8) auf einem beweglichen Träger angeordnet ist, um die Behältnisse (2) an der Strahlungsquelle (6, 7) vorbeizuführen
**dadurch gekennzeichnet, dass**
die Halteelemente (8) zumindest teilweise in das Innere ders Behältnisses (2) eingreifent und so gewährleistet wird, dass bei einer Außensterilisation des Behältnisses (2) Ladungsträger von der Ladungsträgerquelle (6, 7) bzw. der Beschleunigungseinrichtung direkt auf eine Außenseite des Behältnisses (2) geleitet werden können ohne dass das Halteelement (8) oder mindestens ein Abschnitt des Halteelements (8) zwischen der Ladungsträgerquelle (6, 7) bzw. der Beschleunigungseinrichtung und einer Außenseite des Behältnisses (2) angeordnet ist, wobei ein in das Innere des Behältnisses (2) einführbarer Anteil des Halteelements (8) in seiner Dimension zumindest in einer Richtung, welche senkrecht zur Längsrichtung des Behältnisses steht, veränderbar ist.

## Claims

1. An apparatus (1) for the sterilization of containers (2) and, in particular, of an outer wall of containers (2) by means of accelerated charge carriers with a radiation source (6, 7) namely a charge carrier source (6, 7) for generating the charge carriers and with an acceleration device which accelerates the charge carriers, in particular in the direction of an outer wall of the containers, wherein the containers (2) are movable by means of a transport device (19) along a pre-set transport path (5) relative to the charge carrier source (6, 7) and the transport device (19) has a plurality of holding elements (8) arranged on a movable carrier for holding at least one container (2), in order to guide the containers (2) past the radiation source (6, 7),
**characterized in that**
the holding elements (8) are capable of being introduced at least in part into the interior of the containers (2) and of being brought into contact with an inner wall of the containers (2) and a portion of the holding element (8) which can be introduced in the interior of the container (2) can be changed with respect to its dimension at least in a direction perpendicular to the longitudinal direction of the container.

2. An apparatus (1) for the sterilization of containers (2) according to claim 1,
**characterized in that**
the interior of the container (2) is capable of being contacted by the holding element (8) at least in the region of an aperture (15).

3. An apparatus (1) for the sterilization of containers (2) according to claim 1 or 2,
**characterized in that**
the holding element (8) is rotatable, in order to be able to rotate around a container (2) gripped by the holding element (8) with respect to its longitudinal axis (L).

4. An apparatus (1) for the sterilization of containers (2) according to at least one of the preceding claims,
**characterized in that**
the holding element (8) capable of being introduced into the aperture of the containers is a clamping sleeve.

5. An apparatus (1) according to claim 4,
**characterized in that**
the clamping sleeve is designed in such a way that the springing action for gripping the containers is produced solely by the design and/or the choice of material of the clamping sleeve, in such a way that no further components are required for the springing action for gripping and securing the container.

6. An apparatus (1) for the sterilization of containers (2) according to at least one of the preceding claims,
**characterized in that**
the movable carrier (19) is a transport wheel or a transport star wheel.

7. An apparatus (1) for the sterilization of containers (2) according to at least one of the preceding claims,
**characterized in that**
a distance between two adjacent holding elements (8) is variable in a transport direction (T) of the containers.

8. An apparatus (1) for the sterilization of containers (2) according to at least one of the preceding claims,
**characterized in that**
the apparatus (1) comprises a clean room which includes at least one portion of a transport path (5) of the containers (2) and preferably also of the holding elements (8).

9. An apparatus (1) for the sterilization of containers (2) according to at least one of the preceding claims,
**characterized in that**
the holding element (8) is movable in a direction which extends substantially parallel to the longitudinal axis of the containers (2).

10. A plant for the treatment of containers (2), which comprises at least one apparatus (1) for the sterilization of containers (2) according to at least one of the preceding claims,
**characterized in that**
the plant has a further apparatus for the sterilization of an inner wall of the containers.

11. A plant for the treatment of containers (2) according to claim 10,
**characterized in that**
the further apparatus for sterilization comprises at least one holding element by which a container (2) is capable of being brought into contact on the outside thereof, and by means of which plant the inner wall of the container (2) is preferably capable of being acted upon with charge carriers by means of a source - inserted at least in sections into the interior of the container - for accelerated charge carriers.

12. A plant for the treatment of containers (2) according to claim 10,
**characterized in that**
the plant comprises at least one transport element (19), preferably a transport star wheel, which is suitable for taking up a container (2) from an apparatus for the sterilization of containers (2) and transferring it to a further apparatus for sterilization.

13. A plant for the treatment of containers (2) according to any one of claims 10-12,
**characterized in that**
the plant has a device for filling containers (2) and this device is arranged downstream with respect to the apparatus (1) for sterilization.

14. A method of sterilizing containers (2) by means of accelerated charge carriers, which are produced in a radiation source (6, 7), namely a charge carrier source (6, 7) and which are accelerated in the direction of the container (2) by an acceleration device, wherein the containers (2) are moved relative to the charge carrier source (6, 7) by means of a transport device (19) which has a plurality of holding elements (8) for holding the containers (2), wherein it is provided that the plurality of holding elements (8) is arranged on a movable carrier, in order to guide the containers (2) past the radiation source (6, 7),
**characterized in that**
the holding elements (8) engage at least in part in the interior of the containers (2) and so it is ensured that during an external sterilization of the container (2) charge carriers can be guided from the charge carrier source (6, 7) or the acceleration device respectively directly to an external side of the container (2), without the holding element (8) or at least a portion of the holding element (8) being situated between the charge carrier source (6, 7) or the acceleration device respectively and an external side of the container (2), wherein a portion of the holding element (8) which can be introduced in the interior of the container (2) can be changed with respect to its dimension at least in a direction perpendicular to the longitudinal direction of the container.

## Revendications

1. Dispositif (1) pour la stérilisation de récipients (2) et en particulier d'une paroi extérieure de récipients (2) au moyen de porteurs de charge accélérés, comprenant une source de rayonnement (6, 7), à savoir ici une source de porteurs de charge (6, 7) servant à générer les porteurs de charge et un dispositif d'accélération, lequel accélère les porteurs de charge - en particulier en direction d'une paroi extérieure des récipients, les récipients (2) étant déplacés relativement par rapport à la source de porteurs de charge (6, 7) le long d'une voie de transport (5) prédéfinie au moyen d'un dispositif de transport (19) et le dispositif de transport (19) comprenant une pluralité d'éléments de retenue (8) disposés sur un support mobile servant à retenir au moins un récipient (2), pour amener les récipients (2) à la source de rayonnement (6, 7),
**caractérisé en ce que**
les éléments de retenue (8) peuvent être introduits au moins en partie à l'intérieur du récipient (2) et peuvent être amenés en contact avec une paroi intérieure du récipient (2) et la dimension d'une partie de l'élément de retenue (8) pouvant être introduite à l'intérieur du récipient (2) peut être modifiée au moins dans une direction perpendiculaire à la direction longitudinale du récipient.

2. Dispositif (1) de stérilisation de récipients (2) selon la revendication 1,
**caractérisé en ce que**
l'intérieur du récipient (2) peut être en contact avec l'élément de retenue (8) au moins dans la zone d'une embouchure (15).

3. Dispositif (1) de stérilisation de récipients (2) selon la revendication 1 ou 2,
**caractérisé en ce que**
l'élément de retenue (8) est rotatif, afin de pouvoir amener en rotation un récipient (2) saisi par l'élément de retenue (8) autour de son axe longitudinal (L).

4. Dispositif (1) de stérilisation de récipients (2) selon au moins l'une des revendications précédentes,
**caractérisé en ce que**
l'élément de retenue (8) pouvant être introduit dans l'embouchure des récipients est un manchon de serrage.

5. Dispositif (1) selon la revendication 4,
**caractérisé en ce que**
le manchon de serrage est configuré de telle manière que l'effet élastique permettant la préhension des récipients est obtenu uniquement par la conception et/ou le choix de matériau du manchon de serrage, si bien qu'aucun autre élément n'est nécessaire pour obtenir l'effet élastique permettant la préhension et le maintien du récipient.

6. Dispositif (1) de stérilisation de récipients (2) selon au moins l'une des revendications précédentes,
**caractérisé en ce que**
le support (19) mobile est une roue de transport ou un système de transport en étoile.

7. Dispositif (1) de stérilisation de récipients (2) selon au moins l'une des revendications précédentes,
**caractérisé en ce qu'**
un écart entre deux éléments de retenue (8) adjacents dans une direction de transport (T) des récipients peut être modifié.

8. Dispositif (1) de stérilisation de récipients (2) selon au moins l'une des revendications précédentes,
**caractérisé en ce que**
le dispositif (1) comprend une salle blanche, laquelle comprend au moins une partie d'une voie de transport (5) des récipients (2) et de préférence également des éléments de retenue (8).

9. Dispositif (1) de stérilisation de récipients (2) selon au moins l'une des revendications précédentes,
**caractérisé en ce que**
l'élément de retenue (8) est mobile dans une direction qui s'étend sensiblement parallèlement à l'axe longitudinal des récipients (2).

10. Installation de traitement de récipients (2), laquelle comprend au moins un dispositif (1) de stérilisation de récipients (2) selon au moins l'une des revendications précédentes,
**caractérisée en ce que**
l'installation comprend un autre dispositif de stérilisation d'une paroi intérieure des récipients.

11. Installation de traitement de récipients (2) selon la revendication 10,
**caractérisée en ce que**
l'autre dispositif de stérilisation comprend au moins un élément de retenue, avec lequel un récipient (2) peut être amené en contact par son côté extérieur et au moyen duquel de préférence la paroi intérieure du récipient (2) peut être exposée à des porteurs de charge à l'aide d'une source de porteurs de charge accélérés introduite au moins en partie à l'intérieur du récipient.

12. Installation de traitement de récipients (2) selon la revendication 10,
**caractérisée en ce que**
l'installation comprend au moins un élément de transport (19), de préférence un système de transport en étoile, lequel est adapté à réceptionner un récipient (2) d'un dispositif de stérilisation de récipients (2) et à le transférer sur un autre dispositif de stérilisation.

13. Installation de traitement de récipients (2) selon l'une des revendications 10 à 12,
**caractérisée en ce que**
l'installation comprend un système de remplissage de récipients (2) et ce système est agencé en aval par rapport au dispositif (1) de stérilisation pour stériliser.

14. Procédé de stérilisation de récipients (2) au moyen de porteurs de charge accélérés, lesquels sont générés dans une source de rayonnement (6, 7), à savoir ici une source de porteurs de charge (6, 7) et accélérés en direction du récipient (2) à l'aide d'un dispositif d'accélération, les récipients (2) étant déplacés par rapport à la source de porteurs de charge (6, 7) au moyen d'un dispositif de transport (19), lequel comprend une pluralité d'éléments de retenue (8) servant à retenir les récipients (2), la pluralité d'éléments de retenue (8) étant disposée sur un support mobile afin d'amener les récipients (2) à la source de rayonnement (6, 7),
**caractérisé en ce que**
les éléments de retenue (8) s'insèrent au moins en partie à l'intérieur des récipients (2), ce qui permet de garantir que lors d'une stérilisation de l'extérieur du récipient (2), les porteurs de charge provenant de la source de porteurs de charge (6, 7) ou du dispositif d'accélération respectivement puissent être dirigés directement sur un côté extérieur du récipient (2) sans que l'élément de retenue (8) ou au moins une section de l'élément de retenue (8) soit agencé(e) entre la source de porteurs de charge (6, 7) ou le dispositif d'accélération respectivement et un côté extérieur du récipient (2), la dimension de l'élément de retenue (8) pouvant être introduite à l'intérieur du récipient (2) peut être modifiée au moins dans une direction perpendiculaire à la direction longitudinale du récipient.
